# EUROPÄISCHE PATENTSCHRIFT

(11) **EP 1 136 559 B1**
(45) Veröffentlichungstag und Bekanntmachung des Hinweises auf die Patenterteilung: **21.04.2010**
(21) Anmeldenummer: 01103850.2
(22) Anmeldetag: 16.02.2001
(51) Int. Cl.: C12N 15/31, C12N 15/54, C12N 15/63, C12N 15/77, C12N 1/21, C12P 13/04, C12P 13/08

(54) **Für das dapC-Gen kodierende Nukleotidsequenzen und Verfahren zur Herstellung von L-Lysin**
Nucleotide sequences coding for the dapC-gene and process for the preparation of L-lysine
Séquences nucléotidiques codant pour le gène dapC et procédé pour la préparation de L-lysine

(30) Priorität: 23.03.2000 DE 10014546
(43) Veröffentlichungstag der Anmeldung: 26.09.2001
(73) Patentinhaber: Evonik Degussa GmbH, 45128 Essen (DE)
(72) Erfinder: Möckel, Bettina, Dr., 40597 Düsseldorf (DE); Weissenborn, Anke, 72076 Tübingen (DE); Pfefferle, Walter, Dr., 33790 Halle, (Westf.) (DE); Hartmann, Michael, 33607 Bielefeld (DE); Kalinowski, Jörn, Dr., 33615 Bielefeld (DE); Pühler, Alfred, Prof., 33739 Bielefeld (DE)

(56) Entgegenhaltungen:
- EP-A- 1 108 790
- WO-A-01/00843
- DATABASE EMBL [Online] 6. Dezember 1997 (1997-12-06) COLE ET AL.: Database accession no. al010186 XP002256933
- LEDWIDGE RICHARD ET AL: "The dual biosynthetic capability of N-acetylornithine aminotransferase in arginine and lysine biosynthesis" BIOCHEMISTRY, Bd. 38, Nr. 10, 9. März 1999 (1999-03-09), Seiten 3019-3024, XP002256931 ISSN: 0006-2960
- FUCHS THILO M ET AL: "Characterization of a Bordetella pertussis diaminopimelate (DAP) biosynthesis locus identifies dapC, a novel gene coding for an N-succinyl-L,L-DAP aminotransferase" JOURNAL OF BACTERIOLOGY, Bd. 182, Nr. 13, Juli 2000 (2000-07), Seiten 3626-3631, XP002256932 ISSN: 0021-9193
- SAHM H ET AL: "CONSTRUCTION OF L-LYSINE, L-THREONINE-, OR L-ISOLEUCINE-OVERPRODUCING STRAINS OF CORYNEBACTERIUM GLUTAMICUM" ANNALS OF THE NEW YORK ACADEMY OF SCIENCES, NEW YORK ACADEMY OF SCIENCES, NEW YORK, NY, US, Bd. 782, 1996, Seiten 25-39, XP000943151 ISSN: 0077-8923

### SEQUENZPROTOKOLL

<110> Degussa-Huels AG
<120> Für das dapC-Gen kodierende Muklectidsequenzen und Verfahren zur Herstellung von L-Lysin
<130> 990217 BT
<140>
   <141>
<160> 6
<170> PatentIn Ver. 2.1
<210> 1
   <211> 1300
   <212> DNA
   <213> Corynebacterium glutamicum
<220>
   <221> CDS
   <222> (91)..(1191)
   <223> dapC-Gen
<400> 1
   gggctcccca ggtggtgcgg ctaagcttgg accacaagat tttgatcacc caatgatcgc 60
   tgcgctgccg cctcaggcat aatctaacgc atg acc tct cgc acc ccg ctt gtt  114
                                    Met Thr Ser Arg Thr Pro Leu Val
                                      1               5
   tct gtt ctt cct gat ttt ccg tgg gat tcg ctc gct tcc gca aaa gcc   162
   Ser Val Leu Pro Asp Phe Pro Trp Asp Ser Leu Ala Ser Ala Lys Ala
        10                  15                  20
   aaa gct gcg tct cac ccg gat ggg atc gtg aat ctt tct gtt ggc act   210
   Lys Ala Ala Ser His Pro Asp Gly Ile Val Asn Leu Ser Val Gly Thr
    25                  30                  35                  40
   ccg gtt gat ccg gtc gcg ccc agc att cag atc gcg ttg gca gaa gca   258
   Pro Val Asp Pro Val Ala Pro Ser Ile Gln Ile Ala Leu Ala Glu Ala
                    45                  50                  55
   gcg ggg ttt tcg ggt tac cct caa acc atc ggc acc ccg gaa ctc cgc   306
   Ala Gly Phe Ser Gly Tyr Pro Gln Thr Ile Gly Thr Pro Glu Leu Arg
                60                  65                  70
   gca gcc atc agg ggc gcg ctt gag cgg cgc tac aac atg aca aag ctt   354
   Ala Ala Ile Arg Gly Ala Leu Glu Arg Arg Tyr Asn Met Thr Lys Leu
            75                  80                  85
   gtc gac gcc tcc ctc ctc ccc gtc gtg ggt acc aag gag gca att gcc   402
   Val Asp Ala Ser Leu Leu Pro Val Val Gly Thr Lys Glu Ala Ile Ala
        90                  95                 100
   ctt ctt cca ttc gcg ttg ggt att tcc ggc acc gtt gtc atc cca gag   450
   Leu Leu Pro Phe Ala Leu Gly Ile Ser Gly Thr Val Val Ile Pro Glu
   105                 110                 115                 120
   att gcg tac cca acc tac gaa gtc gct gtc gtg gcc gca gga tgc acc     498
   Ile Ala Tyr Pro Thr Tyr Glu Val Ala Val Val Ala Ala Gly Cys Thr
                   125                 130                 135
   gtg ttg cgt tct gat tcg ctg ttt aag ctc ggc ccg cag atc ccg tcg     546
   Val Leu Arg Ser Asp Ser Leu Phe Lys Leu Gly Pro Gln Ile Pro Ser
               140                 145                 150
   atg atg ttt atc aac tca cca tcc aac ccc aca ggc aag gtt ctg ggc     594
   Met Met Phe Ile Asn Ser Pro Ser Asn Pro Thr Gly Lys Val Leu Gly
           155                 160                 165
   atc cca cac ttg cgc aag gtt gtg aag tgg gcg cag gaa aac aac gtg     642
   Ile Pro His Leu Arg Lys Val Val Lys Trp Ala Gln Glu Asn Asn Val
       170                 175                 180
   atc ctc gca gct gat gaa tgc tac ttg ggt ctt ggc tgg gac gat gaa     690
   Ile Leu Ala Ala Asp Glu Cys Tyr Leu Gly Leu Gly Trp Asp Asp Glu
   185                 190                 195                 200
   aac cca ccg atc tca att ttg gat cca cgt gtc tgc gat ggc gac cac     738
   Asn Pro Pro Ile Ser Ile Leu Asp Pro Arg Val Cys Asp Gly Asp His
                   205                 210                 215
   acc aac ttg atc gcc att cac tcg ctg tct aaa acc tca aac ctc gct     786
   Thr Asn Leu Ile Ala Ile His Ser Leu Ser Lys Thr Ser Asn Leu Ala
               220                 225                 230
   tct tac cgc gca ggt tac ctc gtt ggc gat cca gcg ctg att ggt gaa     834
   Ser Tyr Arg Ala Gly Tyr Leu Val Gly Asp Pro Ala Leu Ile Gly Glu
           235                 240                 245
   ctc acg gaa gtc cgt aag aac ttg ggt ctc atg gtt cct ttc cca atc     882
   Leu Thr Glu Val Arg Lys Asn Leu Gly Leu Met Val Pro Phe Pro Ile
       250                 255                 260
   cag cag gcc atg atc gca gcc ctc aac gac gat gac caa gag gca ggg     930
   Gln Gln Ala Met Ile Ala Ala Leu Asn Asp Asp Asp Gln Glu Ala Gly
   265                 270                 275                 280
   cag aag ctc acc tac gcg att cgt cga gca aaa ctc atg cgc gcc ctg     978
   Gln Lys Leu Thr Tyr Ala Ile Arg Arg Ala Lys Leu Met Arg Ala Leu
                   285                 290                 295
   ttg gaa tcc ggc ttt cag gta gat aat tct gaa gcg ggt ctg tac ctc    1026
   Leu Glu Ser Gly Phe Gln Val Asp Asn Ser Glu Ala Gly Leu Tyr Leu
               300                 305                 310
   tgg gcg acg cgt gaa gaa cct tgc cgt gac act gtc gat tgg ttc gct    1074
   Trp Ala Thr Arg Glu Glu Pro Cys Arg Asp Thr Val Asp Trp Phe Ala
           315                 320                 325
   gag cgt ggc att ctc gtt gcc cca gga gac ttc tat ggc cct cgc gga    1122
   Glu Arg Gly Ile Leu Val Ala Pro Gly Asp Phe Tyr Gly Pro Arg Gly
       330                 335                 340
   gcg cag cat gtg cgt gtg gcg atg acc gaa acc gac gag cgc gtc gac    1170
   Ala Gln His Val Arg Val Ala Met Thr Glu Thr Asp Glu Arg Val Asp
   345                 350                 355                 360
   gcc ttt gtt tct cgc ctg agc taaacacgac taagcttatt ttgtttaatt      1221
   Ala Phe Val Ser Arg Leu Ser
                       365
   gagtttgaag ttttccgtcg aaagaggcca tttgagttcc gagtccagtc ctgagtcgag 1281
   taccgagcaa aaaacctgg                                              1300
<210> 2
   <211> 367
   <212> PRT
   <213> Corynebacterium glutamicum
<900> 2
<210> 3
   <211> 1300
   <212> DNA
   <213> Corynebacterium glutamicum
<220>
   <221> CDS
   <222> (91) .. (1191)
   <223> dapC-Allel
<400> 3
   gggctcccca ggtggtgcgg ctaagcttgg accacaagat tttgatcacc caatgatcgc 60
   tgcgctgccg cctcaggcat aatctaacgc atg acc tct cgc acc ccg ctt gtt 114
   Met Thr Ser Arg Thr Pro Leu Val
   1 5
   tct gtt ctt cct gat ttt ccg tgg gat tcg ctc gct tcc gca aaa gcc 162
   Ser Val Leu Pro Asp Phe Pro Trp Asp Ser Leu Ala Ser Ala Lys Ala
   10 15 20
   aaa gct gcg tct cac ccg gat ggg atc gtg aat ctt tct gtt ggc act 210
   Lys Ala Ala Ser His Pro Asp Gly Ile Val Asn Leu Ser Val Gly Thr
   25 30 35 40
   ccg gtt gat ccg gtc gcg ccc agc att cag atc gcg ttg gca gaa gca 258
   Pro Val Asp Pro Val Ala Pro Ser Ile Gln Ile Ala Leu Ala Glu Ala
   45 50 55
   gcg ggg ttt tcg ggt tac cct caa acc atc ggc acc ccg gaa ctc cgc 306
   Ala Gly Phe Ser Gly Tyr Pro Gln Thr Ile Gly Thr Pro Glu Leu Arg
   60 65 70
   gca gcc atc agg ggc gcg ctt gag cgg cgc tac aac atg aca aag ctt    354
   Ala Ala Ile Arg Gly Ala Leu Glu Arg Arg Tyr Asn Met Thr Lys Leu
           75                  80                  85
   gtc gac gcc tcc ctc ctc ccc gtc gtg ggt acc aag gag gca att gcc    402
   Val Asp Ala Ser Leu Leu Pro Val Val Gly Thr Lys Glu Ala Ile Ala
       90                  95                  100
   ctt ctt cca ttc gcg ttg ggt att tcc ggc acc gtt gtc atc cca gag    450
   Leu Leu Pro Phe Ala Leu Gly Ile Ser Gly Thr Val Val Ile Pro Glu
   105                 110                 115                 120
   att gcg tac cca acc tac gaa gtc gct gtc gtg gcc gca gga tgc acc    498
   Ile Ala Tyr Pro Thr Tyr Glu Val Ala Val Val Ala Ala Gly Cys Thr
                   125                 130                 135
   gtg ttg cgt tct gat tcg ctg ttt aag ctc ggc ccg cag atc ccg tcg    546
   Val Leu Arg Ser Asp Ser Leu Phe Lys Leu Gly Pro Gln Ile Pro Ser
               140                 145                 150
   atg atg ttt atc aac tca cca tcc aac ccc aca ggc aag gtt ctg ggc    594
   Met Met Phe Ile Asn Ser Pro Ser Asn Pro Thr Gly Lys Val Leu Gly
           155                 160                 165
   atc cca cac ttg cgc aag gtt gtg aag tgg gcg cag gaa aac aac gtg    642
   Ile Pro His Leu Arg Lys Val Val Lys Trp Ala Gln Glu Asn Asn Val
       170                 175                 180
   atc ctc gca gct gat gaa tgc tac ttg ggt ctt ggc tgg gac gat gaa    690
   Ile Leu Ala Ala Asp Glu Cys Tyr Leu Gly Leu Gly Trp Asp Asp Glu
   185                 190                 195                 200
   aac cca ccg atc tca att ttg gat cta cgt gtc tgc gat ggc gac cac    738
   Asn Pro Pro Ile Ser Ile Leu Asp Leu Arg Val Cys Asp Gly Asp His
                   205                 210                 215
   acc aac ttg atc gcc att cac tcg ctg tct aaa acc tca aac ctc gct    786
   Thr Asn Leu Ile Ala Ile His Ser Leu Ser Lys Thr Ser Asn Leu Ala
               220                 225                 230
   tct tac cgc gca ggt tac ctc gtt ggc gat cca gcg ctg att ggt gaa    834
   Ser Tyr Arg Ala Gly Tyr Leu Val Gly Asp Pro Ala Leu Ile Gly Glu
           235                 240                 245
   ctc acg gaa gtc cgt aag aac ttg ggt ctc atg gtt cct ttc cca atc    882
   Leu Thr Glu Val Arg Lys Asn Leu Gly Leu Met Val Pro Phe Pro Ile
       250                 255                 260
   cag cag gcc atg atc gca gcc ctc aac gac gat gac caa gag gca ggg    930
   Gln Gln Ala Met Ile Ala Ala Leu Asn Asp Asp Asp Gln Glu Ala Gly
   265                 270                 275                 280
   cag aag ctc acc tac gcg att cgt cga gca aaa ctc atg cgc gcc ctg    978
   Gln Lys Leu Thr Tyr Ala Ile Arg Arg Ala Lys Leu Met Arg Ala Leu
                   285                 290                 295
   ttg gaa tcc ggc ttt cag gta gat aat tct gaa gcg ggt ctg tac ctc    1026
   Leu Glu Ser Gly Phe Gln Val Asp Asn Ser Glu Ala Gly Leu Tyr Leu
               300                 305                 310
   tgg gcg acg cgt gaa gaa cct tgc cgt gac act gtc gat tgg ttc gct   1074
   Trp Ala Thr Arg Glu Glu Pro Cys Arg Asp Thr Val Asp Trp Phe Ala
           315                 320                 325
   gag cgt ggc att ctc gtt gcc cca gga gac ttc tat ggc cct cgc gga   1122
   Glu Arg Gly Ile Leu Val Ala Pro Gly Asp Phe Tyr Gly Pro Arg Gly
       330                 335                 340
   gcg cag cat gtg cgt gtg gcg atg acc gaa acc gac gag cgc gtc gac   1170
   Ala Gln His Val Arg Val Ala Met Thr Glu Thr Asp Glu Arg Val Asp
   345                 350                 355                 360
   gcc ttt gtt tct cgc ctg agc taaacacgac taagcttatt ttgtttaatt      1221
   Ala Phe Val Ser Arg Leu Ser
                   365
   gagtttgaag ttttccgtcg aaagaggcca tttgagttcc gagtccagtc ctgagtcgag 1281
   taccgagcaa aaaacctgg                                              1300
<210> 4
   <211> 367
   <212> PRT
   <213> Corynebacterium glutamicum
<400> 4
   Met Thr Ser Arg Thr Pro Leu Val Ser Val Leu Pro Asp Phe Pro Trp
    1               5                  10                  15
   Asp Ser Leu Ala Ser Ala Lys Ala Lys Ala Ala Ser His Pro Asp Gly
               20                  25                  30
   Ile Val Asn Leu Ser Val Gly Thr Pro Val Asp Pro Val Ala Pro Ser
           35                  40                  45
   Ile Gln Ile Ala Leu Ala Glu Ala Ala Gly Phe Ser Gly Tyr Pro Gln
       50                  55                  60
   Thr Ile Gly Thr Pro Glu Leu Arg Ala Ala Ile Arg Gly Ala Leu Glu
   65                  70                  75                  80
   Arg Arg Tyr Asn Met Thr Lys Leu Val Asp Ala Ser Leu Leu Pro Val
                   85                  90                  95
   Val Gly Thr Lys Glu Ala Ile Ala Leu Leu Pro Phe Ala Leu Gly Ile
               100                 105                 110
   Ser Gly Thr Val Val Ile Pro Glu Ile Ala Tyr Pro Thr Tyr Glu Val
           115                 120                 125
   Ala Val Val Ala Ala Gly Cys Thr Val Leu Arg Ser Asp Ser Leu Phe
       130                 135                 140
   Lys Leu Gly Pro Gln Ile Pro Ser Met Met Phe Ile Asn Ser Pro Ser
   145                 150                 155                 160
   Asn Pro Thr Gly Lys Val Leu Gly Ile Pro His Leu Arg Lys Val Val
                   165                 170                 175
   Lys Trp Ala Gln Glu Asn Asn Val Ile Leu Ala Ala Asp Glu Cys Tyr
                   180                 185                 190
   Leu Gly Leu Gly Trp Asp Asp Glu Asn Pro Pro Ile Ser Ile Leu Asp
               195                 200                 205
   Leu Arg Val Cys Asp Gly Asp His Thr Asn Leu Ile Ala Ile His Ser
       210                 215                 220
   Leu Ser Lys Thr Ser Asn Leu Ala Ser Tyr Arg Ala Gly Tyr Leu Val
   225                 230                 235                 240
   Gly Asp Pro Ala Leu Ile Gly Glu Leu Thr Glu Val Arg Lys Asn Leu
                   245                 250                 255
   Gly Leu Met Val Pro Phe Pro Ile Gln Gln Ala Met Ile Ala Ala Leu
               260                 265                 270
   Asn Asp Asp Asp Gln Glu Ala Gly Gln Lys Leu Thr Tyr Ala Ile Arg
           275                 280                 285
   Arg Ala Lys Leu Met Arg Ala Leu Leu Glu Ser Gly Phe Gln Val Asp
       290                 295                 300
   Asn Ser Glu Ala Gly Leu Tyr Leu Trp Ala Thr Arg Glu Glu Pro Cys
   305                 310                 315                 320
   Arg Asp Thr Val Asp Trp Phe Ala Glu Arg Gly Ile Leu Val Ala Pro
                   325                 330                 335
   Gly Asp Phe Tyr Gly Pro Arg Gly Ala Gln His Val Arg Val Ala Met
               340                 345                 350
   Thr Glu Thr Asp Glu Arg Val Asp Ala Phe Val Ser Arg Leu Ser
           355                 360                 365
<210> 5
   <211> 32
   <212> DNA
   <213> Künstliche Sequenz
<220>
   <223> Beschreibung der künstlichen Sequenz: Primer
<220>
   <223> Primer DapC (dCex1)
<400> 5
   gatctagaat tcgcctcagg cataatctaa cg 32
<210> 6
   <211> 32
   <212> DNA
   <213> Künstliche Sequenz
<220>
   <223> Beschreibung der künstlichen Sequenz: Primer
<220>
   <223> Primer DapC (dCexna2)
<400> 6
   gactatcta gacagaggac aaggcaatcg ga 32

### SEQUENZPROTOKOLL

<110> Degussa-Huels AG
<120> Für das dapC-Gen kodierende Nukleotidsequenzen und Verfahren zur Herstellung von L-Lysin
<130> 990217 BT
<140>
   <141>
<160> 6
<170> PatentIn Ver. 2.1
<210> 1
   <211> 1300
   <212> DNA
   <213> Corynebacterium glutamicum
<220>
   <221> CDS
   <222> (91)..(1191)
   <223> dapC-Gen
<400> 1
   gggctcccca ggtggtgcgg ctaagcttgg accacaagat tttgatcacc caatgatcgc 60
   tgcgctgccg cctcaggcat aatctaacgc atg acc tct cgc acc ccg ctt gtt  114
                                    Met Thr Ser Arg Thr Pro Leu Val
                                      1               5
   tct gtt ctt cct gat ttt ccg tgg gat tcg ctc gct tcc gca aaa gcc   162
   Ser Val Leu Pro Asp Phe Pro Trp Asp Ser Leu Ala Ser Ala Lys Ala
        10                  15                  20
   aaa gct gcg tct cac ccg gat ggg atc gtg aat ctt tct gtt ggc act   210
   Lys Ala Ala Ser His Pro Asp Gly Ile Val Asn Leu Ser Val Gly Thr
    25                  30                  35                  40
   ccg gtt gat ccg gtc gcg ccc agc att cag atc gcg ttg gca gaa gca   258
   Pro Val Asp Pro Val Ala Pro Ser Ile Gln Ile Ala Leu Ala Glu Ala
                    45                  50                  55
   gcg ggg ttt tcg ggt tac cct caa acc atc ggc acc ccg gaa ctc cgc   306
   Ala Gly Phe Ser Gly Tyr Pro Gln Thr Ile Gly Thr Pro Glu Leu Arg
                60                  65                  70
   gca gcc atc agg ggc gcg ctt gag cgg cgc tac aac atg aca aag ctt   354
   Ala Ala Ile Arg Gly Ala Leu Glu Arg Arg Tyr Asn Met Thr Lys Leu
            75                  80                  85
   gtc gac gcc tcc ctc ctc ccc gtc gtg ggt acc aag gag gca att gcc   402
   Val Asp Ala Ser Leu Leu Pro Val Val Gly Thr Lys Glu Ala Ile Ala
        90                  95                 100
   ctt ctt cca ttc gcg ttg ggt att tcc ggc acc gtt gtc atc cca gag   450
   Leu Leu Pro Phe Ala Leu Gly Ile Ser Gly Thr Val Val Ile Pro Glu
   105                 110                 115                 120
   att gcg tac cca acc tac gaa gtc gct gtc gtg gcc gca gga tgc acc    498
   Ile Ala Tyr Pro Thr Tyr Glu Val Ala Val Val Ala Ala Gly Cys Thr
                   125                 130                 135
   gtg ttg cgt tct gat tcg ctg ttt aag ctc ggc ccg cag atc ccg tcg    546
   Val Leu Arg Ser Asp Ser Leu Phe Lys Leu Gly Pro Gln Ile Pro Ser
               140                 145                 150
   atg atg ttt atc aac tca cca tcc aac ccc aca ggc aag gtt ctg ggc    594
   Met Met Phe Ile Asn Ser Pro Ser Asn Pro Thr Gly Lys Val Leu Gly
           155                 160                 165
   atc cca cac ttg cgc aag gtt gtg aag tgg gcg cag gaa aac aac gtg    642
   Ile Pro His Leu Arg Lys Val Val Lys Trp Ala Gln Glu Asn Asn Val
       170                 175                 180
   atc ctc gca gct gat gaa tgc tac ttg ggt ctt ggc tgg gac gat gaa    690
   Ile Leu Ala Ala Asp Glu Cys Tyr Leu Gly Leu Gly Trp Asp Asp Glu
   185                 190                 195                 200
   aac cca ccg atc tca att ttg gat cca cgt gtc tgc gat ggc gac cac    738
   Asn Pro Pro Ile Ser Ile Leu Asp Pro Arg Val Cys Asp Gly Asp His
                   205                 210                 215
   acc aac ttg atc gcc att cac tcg ctg tct aaa acc tca aac ctc gct    786
   Thr Asn Leu Ile Ala Ile His Ser Leu Ser Lys Thr Ser Asn Leu Ala
               220                 225                 230
   tct tac cgc gca ggt tac ctc gtt ggc gat cca gcg ctg att ggt gaa    834
   Ser Tyr Arg Ala Gly Tyr Leu Val Gly Asp Pro Ala Leu Ile Gly Glu
           235                 240                 245
   ctc acg gaa gtc cgt aag aac ttg ggt ctc atg gtt cct ttc cca atc    882
   Leu Thr Glu Val Arg Lys Asn Leu Gly Leu Met Val Pro Phe Pro Ile
       250                 255                 260
   cag cag gcc atg atc gca gcc ctc aac gac gat gac caa gag gca ggg    930
   Gln Gln Ala Met Ile Ala Ala Leu Asn Asp Asp Asp Gln Glu Ala Gly
   265                 270                 275                 280
   cag aag ctc acc tac gcg att cgt cga gca aaa ctc atg cgc gcc ctg    978
   Gln Lys Leu Thr Tyr Ala Ile Arg Arg Ala Lys Leu Met Arg Ala Leu
                   285                 290                 295
   ttg gaa tcc ggc ttt cag gta gat aat tct gaa gcg ggt ctg tac ctc    1026
   Leu Glu Ser Gly Phe Gln Val Asp Asn Ser Glu Ala Gly Leu Tyr Leu
               300                 305                 310
   tgg gcg acg cgt gaa gaa cct tgc cgt gac act gtc gat tgg ttc gct    1074
   Trp Ala Thr Arg Glu Glu Pro Cys Arg Asp Thr Val Asp Trp Phe Ala
           315                 320                 325
   gag cgt ggc att ctc gtt gcc cca gga gac ttc tat ggc cct cgc gga    1122
   Glu Arg Gly Ile Leu Val Ala Pro Gly Asp Phe Tyr Gly Pro Arg Gly
       330                 335                 340
   gcg cag cat gtg cgt gtg gcg atg acc gaa acc gac gag cgc gtc gac    1170
   Ala Gln His Val Arg Val Ala Met Thr Glu Thr Asp Glu Arg Val Asp
   345                 350                 355                 360
   gcc ttt gtt tct cgc ctg agc taaacacgac taagcttatt ttgtttaatt      1221
   Ala Phe Val Ser Arg Leu Ser
                   365
   gagtttgaag ttttccgtcg aaagaggcca tttgagttcc gagtccagtc ctgagtcgag 1281
   taccgagcaa aaaacctgg                                              1300
<210> 2
   <211> 367
   <212> PRT
   <213> Corynebacterium glutamicum
<400> 2
   Met Thr Ser Arg Thr Pro Leu Val Ser Val Leu Pro Asp Phe Pro Trp
   1               5                   10                  15
   Asp Ser Leu Ala Ser Ala Lys Ala Lys Ala Ala Ser His Pro Asp Gly
               20                  25                  30
   Ile Val Asn Leu Ser Val Gly Thr Pro Val Asp Pro Val Ala Pro Ser
           35                  40                  45
   Ile Gln Ile Ala Leu Ala Glu Ala Ala Gly Phe Ser Gly Tyr Pro Gln
       50                  55                  60
   Thr Ile Gly Thr Pro Glu Leu Arg Ala Ala Ile Arg Gly Ala Leu Glu
   65                  70                  75                  80
   Arg Arg Tyr Asn Met Thr Lys Leu Val Asp Ala Ser Leu Leu Pro Val
                   85                  90                  95
   Val Gly Thr Lys Glu Ala Ile Ala Leu Leu Pro Phe Ala Leu Gly Ile
               100                 105                 110
   Ser Gly Thr Val Val Ile Pro Glu Ile Ala Tyr Pro Thr Tyr Glu Val
           115                 120                 125
   Ala Val Val Ala Ala Gly Cys Thr Val Leu Arg Ser Asp Ser Leu Phe
       130                 135                 140
   Lys Leu Gly Pro Gln Ile Pro Ser Met Met Phe Ile Asn Ser Pro Ser
   145                 150                 155                 160
   Asn Pro Thr Gly Lys Val Leu Gly Ile Pro His Leu Arg Lys Val Val
                   165                 170                 175
   Lys Trp Ala Gln Glu Asn Asn Val Ile Leu Ala Ala Asp Glu Cys Tyr
               180                 185                 190
   Leu Gly Leu Gly Trp Asp Asp Glu Asn Pro Pro Ile Ser Ile Leu Asp
           195                 200                 205
   Pro Arg Val Cys Asp Gly Asp His Thr Asn Leu Ile Ala Ile His Ser
       210                 215                 220
   Leu Ser Lys Thr Ser Asn Leu Ala Ser Tyr Arg Ala Gly Tyr Leu Val
   225                 230                 235                 240
   Gly Asp Pro Ala Leu Ile Gly Glu Leu Thr Glu Val Arg Lys Asn Leu
                   245                 250                 255
   Gly Leu Met Val Pro Phe Pro Ile Gln Gln Ala Met Ile Ala Ala Leu
               260                 265                 270
   Asn Asp Asp Asp Gln Glu Ala Gly Gln Lys Leu Thr Tyr Ala Ile Arg
           275                 280                 285
   Arg Ala Lys Leu Met Arg Ala Leu Leu Glu Ser Gly Phe Gln Val Asp
       290                 295                 300
   Asn Ser Glu Ala Gly Leu Tyr Leu Trp Ala Thr Arg Glu Glu Pro Cys
   305                 310                 315                 320
   Arg Asp Thr Val Asp Trp Phe Ala Glu Arg Gly Ile Leu Val Ala Pro
                   325                 330                 335
   Gly Asp Phe Tyr Gly Pro Arg Gly Ala Gln His Val Arg Val Ala Met
               340                 345                 350
   Thr Glu Thr Asp Glu Arg Val Asp Ala Phe Val Ser Arg Leu Ser
           355                 360                 365
<210> 3
   <211> 1300
   <212> DNA
   <213> Corynebacterium glutamicum
<220>
   <221> CDS
   <222> (91)..(1191)
   <223> dapC-Allel
<400> 3
   gggctcccca ggtggtgcgg ctaagcttgg accacaagat tttgatcacc caatgatcgc 60
   tgcgctgccg cctcaggcat aatctaacgc atg acc tct cgc acc ccg ctt gtt  114
                                    Met Thr Ser Arg Thr Pro Leu Val
                                      1               5
   tct gtt ctt cct gat ttt ccg tgg gat tcg ctc gct tcc gca aaa gcc   162
   Ser Val Leu Pro Asp Phe Pro Trp Asp Ser Leu Ala Ser Ala Lys Ala
        10                  15                  20
   aaa gct gcg tct cac ccg gat ggg atc gtg aat ctt tct gtt ggc act   210
   Lys Ala Ala Ser His Pro Asp Gly Ile Val Asn Leu Ser Val Gly Thr
    25                  30                  35                  40
   ccg gtt gat ccg gtc gcg ccc agc att cag atc gcg ttg gca gaa gca   258
   Pro Val Asp Pro Val Ala Pro Ser Ile Gln Ile Ala Leu Ala Glu Ala
                    45                  50                  55
   gcg ggg ttt tcg ggt tac cct caa acc atc ggc acc ccg gaa ctc cgc   306
   Ala Gly Phe Ser Gly Tyr Pro Gln Thr Ile Gly Thr Pro Glu Leu Arg
                60                  65                  70
   gca gcc atc agg ggc gcg ctt gag cgg cgc tac aac atg aca aag ctt    354
   Ala Ala Ile Arg Gly Ala Leu Glu Arg Arg Tyr Asn Met Thr Lys Leu
            75                  80                  85
   gtc gac gcc tcc ctc ctc ccc gtc gtg ggt acc aag gag gca att gcc    402
   Val Asp Ala Ser Leu Leu Pro Val Val Gly Thr Lys Glu Ala Ile Ala
        90                  95                 100
   ctt ctt cca ttc gcg ttg ggt att tcc ggc acc gtt gtc atc cca gag    450
   Leu Leu Pro Phe Ala Leu Gly Ile Ser Gly Thr Val Val Ile Pro Glu
   105                 110                 115                 120
   att gcg tac cca acc tac gaa gtc gct gtc gtg gcc gca gga tgc acc    498
   Ile Ala Tyr Pro Thr Tyr Glu Val Ala Val Val Ala Ala Gly Cys Thr
                   125                 130                 135
   gtg ttg cgt tct gat tcg ctg ttt aag ctc ggc ccg cag atc ccg tcg    546
   Val Leu Arg Ser Asp Ser Leu Phe Lys Leu Gly Pro Gln Ile Pro Ser
               140                 145                 150
   atg atg ttt atc aac tca cca tcc aac ccc aca ggc aag gtt ctg ggc    594
   Met Met Phe Ile Asn Ser Pro Ser Asn Pro Thr Gly Lys Val Leu Gly
           155                 160                 165
   atc cca cac ttg cgc aag gtt gtg aag tgg gcg cag gaa aac aac gtg    642
   Ile Pro His Leu Arg Lys Val Val Lys Trp Ala Gln Glu Asn Asn Val
       170                 175                 180
   atc ctc gca gct gat gaa tgc tac ttg ggt ctt ggc tgg gac gat gaa    690
   Ile Leu Ala Ala Asp Glu Cys Tyr Leu Gly Leu Gly Trp Asp Asp Glu
   185                 190                 195                 200
   aac cca ccg atc tca att ttg gat cta cgt gtc tgc gat ggc gac cac    738
   Asn Pro Pro Ile Ser Ile Leu Asp Leu Arg Val Cys Asp Gly Asp His
                   205                 210                 215
   acc aac ttg atc gcc att cac tcg ctg tct aaa acc tca aac ctc gct    786
   Thr Asn Leu Ile Ala Ile His Ser Leu Ser Lys Thr Ser Asn Leu Ala
               220                 225                 230
   tct tac cgc gca ggt tac ctc gtt ggc gat cca gcg ctg att ggt gaa    834
   Ser Tyr Arg Ala Gly Tyr Leu Val Gly Asp Pro Ala Leu Ile Gly Glu
           235                 240                 245
   ctc acg gaa gtc cgt aag aac ttg ggt ctc atg gtt cct ttc cca atc    882
   Leu Thr Glu Val Arg Lys Asn Leu Gly Leu Met Val Pro Phe Pro Ile
       250                 255                 260
   cag cag gcc atg atc gca gcc ctc aac gac gat gac caa gag gca ggg    930
   Gln Gln Ala Met Ile Ala Ala Leu Asn Asp Asp Asp Gln Glu Ala Gly
   265                 270                 275                 280
   cag aag ctc acc tac gcg att cgt cga gca aaa ctc atg cgc gcc ctg    978
   Gln Lys Leu Thr Tyr Ala Ile Arg Arg Ala Lys Leu Met Arg Ala Leu
                   285                 290                 295
   ttg gaa tcc ggc ttt cag gta gat aat tct gaa gcg ggt ctg tac ctc   1026
   Leu Glu Ser Gly Phe Gln Val Asp Asn Ser Glu Ala Gly Leu Tyr Leu
               300                 305                 310
   tgg gcg acg cgt gaa gaa cct tgc cgt gac act gtc gat tgg ttc gct   1074
   Trp Ala Thr Arg Glu Glu Pro Cys Arg Asp Thr Val Asp Trp Phe Ala
           315                 320                 325
   gag cgt ggc att ctc gtt gcc cca gga gac ttc tat ggc cct cgc gga   1122
   Glu Arg Gly Ile Leu Val Ala Pro Gly Asp Phe Tyr Gly Pro Arg Gly
       330                 335                 340
   gcg cag cat gtg cgt gtg gcg atg acc gaa acc gac gag cgc gtc gac   1170
   Ala Gln His Val Arg Val Ala Met Thr Glu Thr Asp Glu Arg Val Asp
   345                 350                 355                 360
   gcc ttt gtt tct cgc ctg agc taaacacgac taagcttatt ttgtttaatt      1221
   Ala Phe Val Ser Arg Leu Ser
                   365
   gagtttgaag ttttccgtcg aaagaggcca tttgagttcc gagtccagtc ctgagtcgag 1281
   taccgagcaa aaaacctgg                                              1300
<210> 4
   <211> 367
   <212> PRT
   <213> Corynebacterium glutamicum
<400> 4
   Met Thr Ser Arg Thr Pro Leu Val Ser Val Leu Pro Asp Phe Pro Trp
   1               5                   10                  15
   Asp Ser Leu Ala Ser Ala Lys Ala Lys Ala Ala Ser His Pro Asp Gly
               20                  25                  30
   Ile Val Asn Leu Ser Val Gly Thr Pro Val Asp Pro Val Ala Pro Ser
           35                  40                  45
   Ile Gln Ile Ala Leu Ala Glu Ala Ala Gly Phe Ser Gly Tyr Pro Gln
       50                  55                  60
   Thr Ile Gly Thr Pro Glu Leu Arg Ala Ala Ile Arg Gly Ala Leu Glu
   65                  70                  75                  80
   Arg Arg Tyr Asn Met Thr Lys Leu Val Asp Ala Ser Leu Leu Pro Val
                   85                  90                  95
   Val Gly Thr Lys Glu Ala Ile Ala Leu Leu Pro Phe Ala Leu Gly Ile
               100                 105                 110
   Ser Gly Thr Val Val Ile Pro Glu Ile Ala Tyr Pro Thr Tyr Glu Val
           115                 120                 125
   Ala Val Val Ala Ala Gly Cys Thr Val Leu Arg Ser Asp Ser Leu Phe
       130                 135                 140
   Lys Leu Gly Pro Gln Ile Pro Ser Met Met Phe Ile Asn Ser Pro Ser
   145                 150                 155                 160
   Asn Pro Thr Gly Lys Val Leu Gly Ile Pro His Leu Arg Lys Val Val
                   165                 170                 175
   Lys Trp Ala Gln Glu Asn Asn Val Ile Leu Ala Ala Asp Glu Cys Tyr
               180                 185                 190
   Leu Gly Leu Gly Trp Asp Asp Glu Asn Pro Pro Ile Ser Ile Leu Asp
           195                 200                 205
   Leu Arg Val Cys Asp Gly Asp His Thr Asn Leu Ile Ala Ile His Ser
       210                 215                 220
   Leu Ser Lys Thr Ser Asn Leu Ala Ser Tyr Arg Ala Gly Tyr Leu Val
   225                 230                 235                 240
   Gly Asp Pro Ala Leu Ile Gly Glu Leu Thr Glu Val Arg Lys Asn Leu
                   245                 250                 255
   Gly Leu Met Val Pro Phe Pro Ile Gln Gln Ala Met Ile Ala Ala Leu
               260                 265                 270
   Asn Asp Asp Asp Gln Glu Ala Gly Gln Lys Leu Thr Tyr Ala Ile Arg
           275                 280                 285
   Arg Ala Lys Leu Met Arg Ala Leu Leu Glu Ser Gly Phe Gln Val Asp
       290                 295                 300
   Asn Ser Glu Ala Gly Leu Tyr Leu Trp Ala Thr Arg Glu Glu Pro Cys
   305                 310                 315                 320
   Arg Asp Thr Val Asp Trp Phe Ala Glu Arg Gly Ile Leu Val Ala Pro
                   325                 330                 335
   Gly Asp Phe Tyr Gly Pro Arg Gly Ala Gln His Val Arg Val Ala Met
               340                 345                 350
   Thr Glu Thr Asp Glu Arg Val Asp Ala Phe Val Ser Arg Leu Ser
           355                 360                 365
<210> 5
   <211> 32
   <212> DNA
   <213> Künstliche Sequenz
<220>
   <223> Beschreibung der künstlichen Sequenz: Primer
<220>
   <223> Primer DapC (dCex1)
<400> 5
   gatctagaat tcgcctcagg cataatctaa cg 32
<210> 6
   <211> 32
   <212> DNA
   <213> Künstliche Sequenz
<220>
   <223> Beschreibung der künstlichen Sequenz: Primer
<220>
   <223> Primer DapC (dCexna2)
<400> 6
   gatctatcta gacagaggac aaggcaatcg ga 32

## Beschreibung

Gegenstand der Erfindung sind für das dapC-Gen kodierende Nukleotidsequenzen und Verfahren zur fermentativen Herstellung von L-Lysin unter Verwendung von coryneformen Bakterien, in denen das dapC-Gen (N-Succinyl-Aminoketopimelat-Transaminase-Gen) überexprimiert wird.

### Stand der Technik

Aminosäuren, insbesondere L-Lysin, finden in der Humanmedizin und in der pharmazeutischen Industrie, insbesondere aber in der Tierernährung, Anwendung.

Es ist bekannt, daß Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenase, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Metabolite sind und L-Aminosäuren wie z.B. L-Lysin produzieren.

Seit einigen Jahren werden ebenfalls Methoden der rekombinanten DNA-Technik zur Stammverbes::erung Aminosäure produzierender Stämme von Corynebacterium eingesetzt, indem man einzelne Aminosäure-Biosynthesegene amplifiziert und die Auswirkung auf die Aminosäure-Produktion untersucht. Obersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)) , Eggeling (Amino Acids 6: 261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von L-Lysin, bereitzustellen.

### Beschreibung der Erfindung

L-Lysin findet in der Humanmedizin, in der pharmazeutischen Industrie und insbesondere in der Tierernährung Anwendung. Es besteht daher ein allgemeines Interesse daran, neue verbesserte Verfahren zur Herstellung von L-Lysin, bereitzustellen.

Wenn im folgenden L-Lysin oder Lysin erwähnt werden, sind damit nicht nur die Base, sondern auch die Salze wie z.B. Lysin-Monohydrochlorid oder Lysin-Sulfat gemeint.

Gegenstand der Erfindung ist ein isoliertes Polynukleotid aus coryneformen Bakterien, enthaltend mindestens eine-Polynukleotidsequenz, ausgewählt aus der Gruppe
a) Polynukleotid, das für ein Polypeptid mit N-succinyl-Aminoketopimelat-Transaminase Aktivität kodiert, das eine Aminosäuresequenz enthält, die zu mindestens 90%. identisch ist mit der Aminosäuresequenz von SEQ ID No. 2 oder
b) Polynukleotid, das komplementär ist zu den Polynukleotiden von a).

Gegenstand der Erfindung, ist ebenfalls das Polynukleotid gemäß Anspruch 1, wobei es sich bevorzugt um eine replizierbare DNA handelt, enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

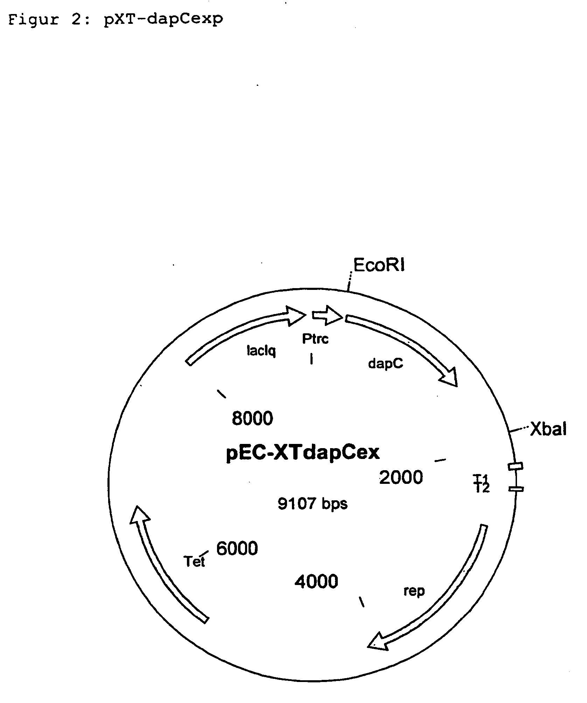

Weitere Gegenstände der Erfindung sind
ein Polynukleotid gemäß Anspruch 5, enthaltend die Nukleotidsequenz wie in SEQ ID No. 1 dargestellt,
ein Polynukleotid, das für ein Polypeptid kodiert, das die Aminosäuresequenz, wie in SEQ ID No. 2 dargestellt, enthält,
ein Vektor, enthaltend das Polynukleotid gemäß Anspruch 1, insbesondere Pendelvektor oder der Plasmidvektor pXT-dapCexp, der in Figur 2 dargestellt und-hinterlegt ist unter der DSM 13254 in DSM 5715.
und als Wirtszelle dienende coryneforme Bakterien, die den Vektor enthalten.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthalten, mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Erfindung sind als Hybridisierungs-Sonden für RNA, cDNA und DNA geeignet, um cDNA in voller Länge zu isolieren, die N-Succinyl-Aminoketopimelat-Transaminase kodieren und solche cDNA oder Gene zu isolieren, die eine hohe Ähnlichkeit der Sequenz mit der des N-Succinyl-Aminoketopimelat-Transaminase Gens aufweisen.

Polynukleotidsequenzen gemäß der Erfindung sind weiterhin geeignet als Primer zur Herstellung von DNA von Genen, die für N-Succinyl-Aminoketopimelat-Transaminase kodieren, durch die Polymerase-Kettenreaktion (PCR).

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Nukleotiden.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß SEQ ID No. 2, insbesondere solche mit der biologischen Aktivität der N-succinyl-Aminoketopimelat-Transaminase und auch solche ein, die zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von L-Lysin, unter Verwendung von coryneformen Bakterien, die insbesondere bereits eine Aminosäure produzierten, und in denen die für das dapC-Gen kodierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw, der Gene erhöht, einen starken Promotor verwendet oder ein Gen oder Allel verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Aminosäuren, insbesondere L-Lysin, aus Glucose, Saccharose, Lactose, Fructose, Maltose, Melasse, Stärke, Cellulöse oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind die zum Beispiel bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464
Corynebacterium glutamicum DSM5715
Corynebacterium glutamicum DSM12866 und
Corynebacterium glutamicum DM58-1.

Den Erfindern gelang es, das neue, für das Enzym N-Succinyl-Aminoketopimelat-Transaminase (EC 2.6.1.17) kodierende dapC-Gen von C. glutamicum zu isolieren.

Zur Isolierung des dapC-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326 (1992)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z.B. bei Sanger et al. (Proceedings of the National Academy of Sciences of the United States of America, 74:5463-5467, 1977) beschrieben ist.

Auf diese Weise wurde die neue für das Gen dapC kodierende DNA-Sequenz von C. glutamicum erhalten, die als SEQ ID NO 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID NO 2 ist die sich ergebende Aminosäuresequenz des dapC-Genproduktes dargestellt.

Kodierende DNA-Sequenzen, die sich aus SEQ ID NO 1 durch die Degeneriertheit des genetischen Kodes ergeben, sind ebenfalls Bestandteil der Erfindung. In gleicher Weise sind DNA-Sequenzen, die mit SEQ ID NO 1 oder Teilen von SEQ ID NO 1 hybridisieren, Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d.h. funktionsneutral sind. Weiterhin ist bekannt, daß Änderungen am N- und/odern C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2 ergeben, sind ebenfalls Bestandteil der Erfindung.

In gleicher Weise sind DNA-Sequenzen, die mit SEQ ID NO 1 oder Teilen von SEQ ID NO 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID NO 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Roche Diagnostics GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Überexpression des dapC-Gens in verbesserter Weise L-Lysin, produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Lysin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP 0 472 869, in US 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in WO 96/15246, bei Malumbres et al. (Gene 134, 15-24 (1993)), in JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispielhaft wurde das erfindungsgemäße dapC-Gen mit Hilfe von Plasmiden überexprimiert.

Als Plasmide eignen sich solche, die in coryneformen Bakterien repliziert werden. Zahlreiche bekannte Plasmidvektoren wie z.B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z.B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Ein Beispiel für ein Plasmid, mit Hilfe dessen das dapC-Gen überexprimiert werden kann, ist der E.coli-C.glutamicum Pendelvektor (shuttle vector) pXT-dapCexp. Er enthält die Replikationsregion rep des Plasmids pGA1 einschließlich des Replikationseffectors per (US-A- 5,175,108; Nesvera et al., Journal of Bacteriology 179, 1525-1532 (1997)), das Tetracyclinresistenz vermittelnde tetA(Z)-Gen des Plasmids pAG1 (US-A- 5,158,891; Genbank-Eintrag beim National Center for Biotechnology Information (NCBI, Bethesda, MD, USA) mit der accession number AF121000), sowie den Replikationsursprung, den trc-Promotor, die Terminationsregionen T1 und T2 und das lacl^{q}-Gen (Repressor des lac-Operons von E.coli) des Plasmids pTRC99A (Amann et al. (1988), Gene 69: 301-315).

Der Pendelvektor pXT-dapCexp ist in Figur 2 dargestellt.

Weiterhin eignen sich solche Plasmidvektoren, mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen bespielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (11994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1999)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm mindestens zwei Kopien des betreffenden Gens.

Weiterhin wurde gefunden, daß durch Austausch der Aminosäure L-Prolin an Position 209 des N-Succinyl-Aminoketopimelat-Transaminase Enzymproteins (siehe SEQ ID No. 2) gegen jede andere proteinogene Aminosäure, insbesondere L-Leucin (Siehe SEQ ID No. 4), mit Ausnahme L-Prolin, eine Verstärkung eintritt und coryneforme Bakterien die den entsprechenden Aminosäureaustausch tragen in verbesserter Weise L-Lysin produzieren. Der Austausch von L-Prolin gegen L-Leucin an Position 209 der Aminosäuresequenz kann vorzugsweise durch den Austausch der Nukleobase Cytosin an Position 716 gegen Thymin erfolgen wie in der Nukleotidsequenz gemäß SEQ ID No. 3 dargestellt.

Für die Mutagenese können klassische Mutageneseverfahren unter Verwendung mutagener Stoffe wie beispielsweise N-Methyl-N'-Nitro-N-Nitrosoguanidin oder ultraviolettes Licht verwendet werden. Weiterhin können für die Mutagenese invitro Methoden wie beispielsweise eine Behandlung mit Hydroxylamin (Molecular and General Genetics 145, 101 pp (1978)) oder mutagene Oligonukleotide (T. A. Brown: Gentechnologie für Einsteiger, Spektrum Akademischer Verlag, Heidelberg, 1993) oder die Polymerasekettenreaktion (PCR), wie sie im Handbuch von Newton und Graham (PCR, Spektrum Akademischer Verlag, Heidelberg, 1994) beschrieben ist, verwendet werden.

Gegenstand der Erfindung sind dementsprechend weiterhin coryneforme Bakterien die N-Succinyl-Aminoketopimelat-Transaminase Enzymproteine enthalten bei denen die unter SEQ ID No. 2 gezeigte Aminosäuresequenz an Position 209 gegen eine andere Aminosäure ausgenommen L-Prolin ausgetauscht ist. Ein weiterer Aspekt dieser Erfindung sind coryneforme Bakterien, die ein entsprechendes Enzymprotein enthalten, in dem die Aminosäure L-Prolin an Position 209 des Enzymproteins (siehe SEQ ID No. 2) gegen L-Leucin (Siehe SEQ ID No. 4), ausgetauscht ist.

Ein weiterer Gegenstand der Erfindung sind dementsprechend aus coryneformen Bakterien stammende Polynukleotidsequenzen, die Gene enthalten, die für die genannten N-Succinyl-Aminoketopimelat-Transaminase Enzymproteine kodieren.

Noch ein Gegenstand dieser Erfindung sind coryneforme Bakterien, die eine für ein N-Succinyl-Aminoketopimelat-Transaminase Enzymprotein, das durch den Austausch von L-Prolin gegen L-Leucin an Position 209 gekennzeichnet ist, kodierende DNA enthalten, welche anstelle der Nukleobase Cytosin an Position 716 (Siehe SEQ ID No. 1) Thymin enthält wie in SEQ ID No. 3 dargestellt.

Zusätzlich kann es für die Produktion von L-Lysin vorteilhaft sein, neben dem dapC-Gen eines oder mehrere Enzyme des jeweiligen Biosyntheseweges, der Glykolyse, der Anaplerotik oder des Aminosäure-Exports zu verstärken oder zu überexprimieren.

So kann beispielsweise zusätzlich zum dapC-Gen für die Herstellung von L-Lysin gleichzeitig eines oder mehrere der Gene, ausgewählt aus der Gruppe
- das für eine feed back resistente Aspartatkinase kodierende lysC-Gen (Kalinowski et al. (1990), Molecular and General Genetics 224: 317-324),
- das für die Aspartat-Semialdehyd Dehydrogenase kodierende asd-Gen (EP-A 0 219 027; Kalinowski et al. (1991), Molecular Microbiology 5:1197-1204, und Kalinowski et al. (1991), Molecular and General Genetics 224: 317-324),
- das für die Dihydrodipicolinat-Synthase kodierende dapA-Gen (EP-B 0 197 335),
- das für die Dihydrodipicolinat-Reduktase kodierende dapB-Gen (Genbankeintrag accession number X67737; Pisabarro et al. (1993), Journal of Bacteriology, 175(9): 2743-2749),
- das für die Tetrahydrodipicolinat-Succinylase kodierende dapD-Gen (Genbankeintrag accession number AJ004934; Wehrmann et al. (1998), Journal of Bacteriology 180: 3159-3163),
- das für die N-Succinyl-Diaminopimelat-Desuccinylase kodierende dapE-Gen (Genbankeintrag accession number X81379; Wehrmann et al. (1994), Microbiology 140: 3349-3356),
- das für die Diaminopimelat-Epimerase kodierende dapF-Gen (DE: 199 43 587.1, DSM12968),
- das für die Diaminopimelat-Decarboxylase kodierende lysA-Gen (Genbankeintrag accession number X07563; Yeh et al. (1988), Molecular and General Genetics 212: 112-119),
- das für die Diaminopimelat-Dehydrogenase kodierende ddh-Gen (Ishino et al. (1988), Agricultural and Biological Chemistry 52(11): 2903-2909)
- das für den Lysin-Export kodierende lysE-Gen (DE-A-195 48 222),
- das für die Pyruvat Carboxylase kodierende pyc-Gen (Eikmanns (1992), Journal of Bacteriology 174: 6076-6086),
- das für die Malat-Chinon-Oxidoreduktase kodierende mqo-Gen (Molenaar et al. (1998), European Journal of Biochemistry 254: 395-403),
- das zwa1-Gen (DE: 19959328.0, DSM 13115).
- das für die Glutamatdehydrogenase kodierende gdh-Gen (Börmann et al. (1992), Molecular Microbiology 6, 317-326)
gleichzeitig überexprimiert oder amplifiziert werden. Die gleichzeitige Verstärkung eines oder mehrerer Gene, ausgewählt aus der Kruppe dapD, dapE und dapF wird bevorzugt.

Weiterhin kann es für die Produktion von L-Lysin, vorteilhaft sein, zusätzlich zur Verstärkung des dapC-Gens, gegebenenfalls in Kombination mit einem oder mehreren Genen ausgewählt aus der Gruppe dapD, dapE und dapF, gleichzeitig
- das für die Phosphoenolpyruvat-Carboxykinase kodierende pck-Gen (DE 199 50 409.1, DSM 13047), oder
- das für die Glucose-6-Phosphat Isomerase kodierende pgi-Gen (US 09/396,478, DSM 12969), oder
- das für die Pyruvat-Oxidase kodierende poxB Gen (DE: 19951975.7, DSM 13114), oder
- das zwa2-Gen (DE: 19959327.2, DSM 13113:, oder
- die für die Succinyl-CoA Synthetase kodierenden Gene sucC oder sucD (DE: 19956686.0)
abzuschwächen.

Weiterhin kann es für die Produktion von L-Lysin, vorteilhaft sein, zusätzlich zur Verstärkung des dapC-Gens, gegebenenfalls in Kombination mit einem oder mehreren Genen ausgewählt aus der Gruppe dapD, dapE und dapF, unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) zum Zwecke der Produktion von L-Lysin kultiviert werden. Eine Zusammenfassung über bekannte Kultivierungsmethoden sind im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1999)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und Cellulose, Öle und Fette wie z.B. Sojaöl, Sonnenblumenöl, Erdnußöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an Lysin gebildet hat. Dieses Ziel wird normalerweise innerhalb von 10 Stunden bis 160 Stunden erreicht.

Die Analyse von L-Lysin kann durch Anionenaustauschchromatographie mit anschließender Ninhydrin Derivatisierung erfolgen, so wie bei Spackman et al. (Analytical Chemistry, 30, (1958), 1190) beschrieben.

Folgender Mikroorganismus wurde bei der Deutschen Sammlung für Mikrorganismen und Zellkulturen (DSMZ, Braunschweig, Deutschland) gemäß Budapester Vertrag hinterlegt:
- Corynebacterium glutamicum Stamm DSM5715/pXT-dapCexp als DSM 13254;
- Corynebacterium glutamicum Stamm DSM5715/pJC1dapF als DSM 13382;
- Corynebacterium glutamicum Stamm DSM5715/pJC1dapDE als DSM 13383;
- Corynebacterium glutamicum Stamm DSM5715/pJC1dapDEF als DSM 13384.

Das erfindungsgemäße Verfahren dient zur fermentativen Herstellung von L-Lysin.

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Diagnostics GmbH, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC 13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 mg/l Ampicillin ausplattiert wurden. Nach Inkubation über Nacht bei 37°C wurden rekombinante Einzelklone selektioniert.

### Beispiel 2

### Isolierung und Sequenzierung des dapC-Gens

Die Cosmid-DNA einer Einzelkolonie wurde mit dem Qiaprep Spin Miniprep Kit (Product No. 27106, Qiagen, Hilden, Deutschland) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Diagnostics GmbH, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Deutschland). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 mg/l Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academies of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Deutschland) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZerol-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz des dapC-Gens ist in SEQ ID No. 1 dargestellt. Die Analyse der Nukleotidsequenz ergab ein offenes Leseraster von 1101 Basenpaaren, welches als dapC-Gen bezeichnet wurde. Das dapC-Gen kodiert für ein Polypeptid von 367 Aminosäuren, welches in SEQ ID No. 2 dargestellt ist.

### Beispiel 3

### Herstellung eines Shuttlevektors pXT-dapCexp zur Verstärkung des dapC-Gens in C. glutamicum

### 3.1. Klonierung des dapC Gens

Aus dem Stamm ATCC 13032 nach der Methode von Eikmanns et al. (Microbiology 140: 1817-1828 (1994)) chromosomale DNA isoliert. Aufgrund der aus Beispiel 2 für C. glutamicum bekannten Sequenz des dapC Gens wurden die folgenden Oligonukleotide für die Polymerase Kettenreaktion ausgewählt (Siehe auch SEQ ID No. 5 und 6):
DapC (dCex1) :
   5' GAT CTA (GAA TTC) GCC TCA GGC ATA ATC TAA CG 3'
DapC (dCexna2):
   5' GAT CTA (TCT AGA) CAG AGG ACA AGG CAA TCG GA 3'

Die dargestellten Primer wurden von der Firma ARK Scientific GmbH Biosystems (Darmstadt, Deutschland) synthetisiert und nach der Standard-PCR-Methode von Innis et al. (PCR Protocols. A Guide to Methods and Applications, 1990, Academic Press) mit Pwo-Polymerase der Firma Roche Diagnostics GmbH (Mannheim, Deutschland) die PCR Reaktion durchgeführt. Mit Hilfe der Polymerase-Kettenreaktion ermöglichen die Primer die Amplifikation eines ca. 1,6 kb großen DNA-Fragmentes, welches das dapC Gen trägt. Außerdem enthälten der Primer DapC (dCex1)die Sequenz für die Schnittstelle der Restriktionsendonuklease EcoRI, und der Primer DapC (dCexna2) die Schnittstelle der Restriktionsendonuklease XbaI, die in der oben dargestellten Nukleotidabfolge durch Klammern markiert sind.

Das amplifizierte DNA Fragment von ca. 1,6 kb, welches das dapC Gen trägt, wurde mit dem Zero Blunt^{™} Kit der Firma Invitrogen Corporation(Carlsbad, CA, USA; Katalog Nummer K2700-20) in den Vektor pCR®Blunt II(Bernard et al., Journal of Molecular Biology, 234: 534-541 (1993)) ligiert. Anschließend wurde der E. coli Stamm Top10 mit dem Ligationsansatz nach Angaben des Kit-Herstellers (Firma Invitrogen Corporation,Carlsbad, CA, USA) transformiert. Die Selektion von Plasmid-tragenden Zellen erfolgte durch Ausplattieren des Transformationsansatzes auf LB Agar (Sambrook et al., Molecular Cloning: A Laboratory Manual, 2nd Ed., Cold Spring Harbor Laboratory Press, Cold Spring Harbor, N.Y., 1989), der mit 25 mg/l Kanamycin supplementiert worden war. Plasmid-DNA wurde aus einer Transformante mit Hilfe des QIAprep Spin Miniprep Kit der Firma Qiagen (Hilden, Deutschland) isoliert und durch Behandlung mit den Restriktionsenzymen XbaI und EcoRI mit anschließender Agarosegel-Elektrophorese (0,8%) überprüft. Die DNA Sequenz des amplifizierten DNA Fragmentes wurde durch Sequenzierung überprüft. Das Plasmid wurde pCRdapC genannt. Der Stamm wurde als E. coli Top10 / pCRdapC bezeichnet.

### 3.2. Herstellung des E. coli - C. glutamicum Shuttle Vektors pEC-XT99A

Als Ausgangsvektor zur Konstruktion des E. coli-C. glutamicum-Shuttle-Expressionsvektors pEC-XT99A wurde der E.coli-Expressionsvektor pTRC99A (Amann et al. 1988, Gene 69:301-315) verwandt. Nach BspHI-Restriktionsspaltung (Roche Diagnostics GmbH, Mannheim, Deutschland, Produktbeschreibung BspHI, Product No. 1467123) und anschließender Klenow-Behandlung (Amersham Pharmacia Biotech, Freiburg, Deutschland, Produktbeschreibung Klenow Fragment of DNA Polymerase I, Product No. 27-0928-01; Methode nach Sambrook et al., 1989, Molecular Cloning: A Laboratory Manual, Cold Spring Harbor) wurde das Ampicillin-Resistenzgen (bla) gegen das Tetracyclin-Resistenzgen des C. glutamicum Plasmids pAG1 (GenBank Accession No. AF121000) ausgetauscht. Hierzu wurde der Resistenzgen-tragende Bereich als AluI-Fragment (Amersham Pharmacia Biotech, Freiburg, Deutschland, Produktbeschreibung AluI, Product No. 27-0884-01) in den linearisierten E.coli-Expressionsvektor pTRC99A kloniert. Die Ligation wurde wie von Sambrook et al. (1989, Molecular Cloning: A Laboratory Manual, Cold Spring Harbor) beschrieben durchgeführt, wobei das DNA-Gemisch mit T4-Ligase (Amersham Pharmacia Biotech, Freiburg, Deutschland, Produktbeschreibung T4-DNA-Ligase, Product No. 27-0870-04) über Nacht inkubiert wurde. Dieses Ligationsgemisch wurde anschließend in den E. coli-Stamm DH5αmcr (Grant, 1990, Proceedings of the National Academy of Sciences U.S.A., 87:4645-4649) elektroporiert (Tauch et al. 1994, FEMS Microbiology Letters, 123:343-7). Der konstruierte E. coli-Expressionsvektor wurde mit pXT99A bezeichnet.

Als Basis zur Klonierung eines Minimalreplikons aus Corynebacterium glutamicum wurde das Plasmid pGA1 (Sonnen et al. 1991, Gene, 107:69-74) verwandt. Durch BalI/PstI-Restriktionsspaltung (Promega GmbH, Mannheim, Deutschland, Produktbeschreibung BalI, Product No. R6691; Amersham Pharmacia Biotech, Freiburg, Deutschland Produktbeschreibung PstI, Product No. 27-0976-01) des Vektors pGA1 konnte ein 3484 bp großes Fragment in den mit SmaI und PstI (Amersham Pharmacia Biotech, Freiburg, Deutschland, Produktbeschreibung SmaI, Product No. 27-0942-02, Produktbeschreibung PstI, Product No. 27-0976-01) fragmentierten Vektor pK18mob2 (Tauch et al., 1998, Archives of Microbiology 169:303-312) kloniert werden.

Mittels BamHI/XhoI-Restriktionsspaltung (Amersham Pharmacia Biotech, Freiburg, Deutschland, Produktbeschreibung BamHI, Product No. 27-086803, Produktbeschreibung XhoI, Product No. 27-0950-01) und anschließender Klenow-Behandlung (Amersham Pharmacia Biotech, Freiburg, Deutschland, Produktbeschreibung Klenow Fragment of DNA Polymerase I, Product No. 27-0928-01; Methode nach Sambrook et al., 1989, Molecular Cloning: A Laboratory Manual, Cold Spring Harbor) wurde ein 839 bp großes Fragment deletiert. Aus dem mit T4-Ligase (Amersham Pharmacia Biotech, Freiburg, Deutschland, Produktbeschreibung T4-DNA-Ligase, Product No. 27-0870-04) religierten Konstrukt konnte das C. glutamicum Minimalreplikon als 2645 bp großes Fragment in den E.coli-Expressionsvektor pXT99A kloniert werden. Hierzu wurde die DNA des Minimalreplikon-tragenden Konstruktes mit den Restriktionsenzymen KpnI (Amersham Pharmacia Biotech, Freiburg, Deutschland, Produktbeschreibung KpnI, Product No. 27-0908-01) und PstI (Amersham Pharmacia Biotech, Freiburg, Deutschland, Produktbeschreibung PstI, Product No. 27-0886-03) gespalten und anschließend mittels Klenow-Polymerase (Amersham Pharmacia Biotech, Freiburg, Deutschland, Produktbeschreibung Klenow Fragment of DNA Polymerase I, Product No. 27-0928-01) eine 3'-5'-Exonukleasebehandlung (Sambrook et al., 1989, Molecular Cloning: A Laboratory Manual, Cold Spring Harbor) durchgeführt.

In einem parallelen Ansatz wurde der E.coli-Expressionsvektor pXT99A mit dem Restriktionsenzym RsrII (Roche Diagnostics, Mannheim, Deutschland, Produktbeschreibung RsrII, Product No. 1292587) gespalten und mit Klenow-Polymerase (Amersham Pharmacia Biotech, Freiburg, Deutschland, Klenow Fragment of DNA Polymerase I, Product No. 27-0928-01) zur Ligation vorbereitet. Die Ligation des Minimalreplikons mit dem Vektorkonstrukt pXT99A wurde wie von Sambrook et al. (1989, Molecular Cloning: A Laboratory Manual, Cold Spring Harbor) beschrieben durchgeführt, wobei das DNA-Gemisch mit T4-Ligase (Amersham Pharmacia Biotech, Freiburg, Deutschland, Produktbeschreibung T4-DNA-Ligase, Product No. 27-0870-04) über Nacht inkubiert wurde.

Der so konstruierte E. coli-C. glutamicum-Shuttle-Expressionsvektor pEC-XT99A wurde mittels Elektroporation (Liebl et al., 1989, FEMS Microbiology Letters, 53:299-303) in C. glutamicum DSM5715 transferiert. Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 5 mg/l Tetracyclin supplementiert worden war. Die Inkubation erfolgte für 2 Tage bei 33°C.

Plasmid DNA wurde aus einer Transformante nach den üblichen Methoden isoliert (Peters-Wendisch et al., 1998, Microbiology, 144, 915 - 927), mit der Restriktionsendonuklease HindIII geschnitten und das Plasmid durch anschließende Agarosegel-Elektrophorese überprüft.

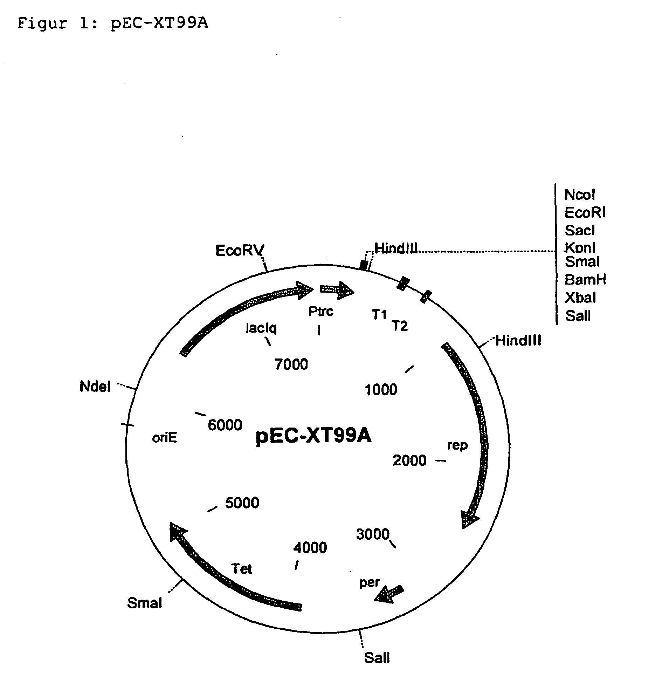

Das so erhaltene Plasmidkonstrukt wurde als pEC-XT99A bezeichnet und ist in Figur 1 dargestellt. Der durch Elektroporation des Plasmides pEC-XT99A in den Corynebacterium glutamicum-Stamm DSM5715 erhaltene Stamm wurde DSM5715/pEC-XT99A genannt und als DSM12967 bei der Deutschen Sammlung für Mikroorganismen und Zellkulturen (DSMZ, Braunschweig, Deutschland) gemäß Budapester Vertrag hinterlegt.

### 3.3. Klonierung von dapC im E. coli-C. glutamicum Shuttle Vektor pEC-XT99A

Als Vektor wurde der in Beispiel 3.2 beschriebene E. coli - C. glutamicum Shuttle-Vektor pEC-XT99A verwendet. DNA dieses Plasmids wurde mit den Restriktionsenzymen EcoRI und XbaI vollständig gespalten und anschließend mit shrimp alkalischer Phosphatase (Roche Diagnostics GmbH, Mannheim, Deutschland, Produktbeschreibung SAP, Product No. 1758250) dephosphoryliert.

Aus dem in Beispiel 3.1. beschriebenen Plasmid pCRdapC wurde das dapC Gen durch vollständige Spaltung mit den Enzymen EcoRI und XbaI isoliert. Das ca 1600bp große dapC Fragment wurde aus dem Agarosegel mit dem QiaExII Gel Extraction Kit (Product No. 20021, Qiagen, Hilden, Germany) isoliert.

Das auf diese Weise gewonnene dapC-Fragment wurde mit dem vorbereiteten Vektor pEC-XT99A gemischt und der Ansatz mit T4-DNA-Ligase (Amersham Pharmacia, Freiburg, Deutschland, Produktbeschreibung T4-DNA-Ligase, Code no.27-0870-04) behandelt. Der Ligationsansatz wurde in den E. coli Stamm DH5α (Hanahan, In: DNA Cloning. A Practical Approach. Vol. I, IRL-Press, Oxford, Washington DC, USA) transformiert. Die Selektion von Plasmid-tragenden Zellen erfolgte durch Ausplattieren des Transformationsansatzes auf LB-Agar (Lennox, 1955, Virology, 1:190) mit 5 mg/l Tetracyclin. Nach Inkubation über Nacht bei 37°C wurden rekombinante Einzelklone selektioniert. Plasmid DNA wurde aus einer Transformante mit dem Qiaprep Spin Miniprep Kit (Product No. 27106, Qiagen, Hilden, Germany) nach Herstellerangaben isoliert und mit den Restriktionsenzymen EcoRI und XbaI gespalten, um das Plasmid durch anschließende Agarosegel-Elektrophorese zu überprüfen. Das erhaltene Plasmid wurde pXT-dapCexp genannt. Es ist in Figur 2 dargestellt.

### Beispiel 4

### Transformation des Stammes DSM5715 mit dem Plasmid pXT-dapCexp

Der Stamm DSM5715 wurde mit dem Plasmid pXT-dapCexp unter Anwendung der von Liebl et al., (FEMS Microbiology Letters, 53:299-303 (1989)) beschriebenen Elektroporationsmethode transformiert. Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 5 mg/l Tetracyclin supplementiert worden war. Die Inkubation erfolgte für 2 Tage bei 33°C.

Plasmid DNA wurde aus einer Transformante nach den üblichen Methoden isoliert (Peters-Wendisch et al., 1998, Microbiology, 144, 915 - 927), mit den Restriktionsendonukleasen EcoRI und XbaI geschnitten und das Plasmid durch anschließende Agarosegel-Elektrophorese überprüft. Der erhaltene Stamm wurde DSM5715/pXT-dapCexp genannt und als DSM 13254 bei der Deutschen Sammlung für Mikrorganismen und Zellkulturen (DSMZ, Braunschweig, Deutschland) gemäß Budapester Vertrag hinterlegt.

### Beispiel 5

### Herstellung von Lysin

Der in Beispiel 4 erhaltene C. glutamicum Stamm DSM5715/pXT-dapCexp wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz-Agar mit Tetracyclin (5 mg/l)) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet.

**Medium Cg III**

| | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |

Der pH-Wert wurde auf pH 7.4
eingestellt

Diesem wurde Tetracyclin (5 mg/l) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,1 betrug. Für die Hauptkultur wurde das Medium MM verwendet.

**Medium MM**

| | |
|---|---|
| CSL (Corn Steep Liquor) | 5 g/l |
| MOPS (Morpholinopropansulfonsäure) | 20 g/l |
| Glucose (getrennt autoklaviert) | 50 g/l |
| (NH₄)₂SO₄ | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| L-Leucin (sterilfiltriert) | 0,1 g/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Tetracyclin (5 mg/l) zugesetzt. Die Kultivierung erfolgte bei 33°C und 80% Luftfeuchte.

Nach 72 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD (660nm) | Lysin-HCl g/l |
|---|---|---|
| DSM5715 | 7,0 | 13,7 |
| DSM5715/pXT-dapCexp | 7,1 | 14,7 |

Folgende Figuren sind beigefügt:
Figur 1: Karte des Plasmids pEC-XT99A
Figur 2: Karte des Plasmids pXT-dapCexp

Die verwendeten Abkürzungen und Bezeichnungen haben folgende Bedeutung.

| | |
|---|---|
| per: | Gen zur Kontrolle der Kopienzahl aus pGA1 |
| oriE: | Plasmidkodierter Replikationsursprung von E. coli |
| rep: | Plasmidkodierter Replikationsursprung aus C. glutamicum Plasmid pGA1 |
| Ptrc: | trc-Promotor aus pTRC99A |
| T1, T2: | Terminatorregionen 1 und 2 aus pTRC99A |
| lacIq: | Repressor-Gen des Lac-Operon |
| Tet: | Resistenzgen für Tetracyclin |
| dapC: | dapC-Gen von C.glutamicum |
| EcoRI: | Schnittstelle des Restriktionsenzyms EcoRI |
| EcoRV: | Schnittstelle des Restriktionsenzyms EcoRV |
| HindIII: | Schnittstelle des Restriktionsenzyms HindIII |
| KpnI: | Schnittstelle des Restriktionsenzyms KpnI |
| SalI: | Schnittstelle des Restriktionsenzyms SalI |
| SmaI: | Schnittstelle des Restriktionsenzyms SmaI |
| NdeI: | Schnittstelle des Restriktionsenzyms NdeI |
| BamHI: | Schnittstelle des Restriktionsenzyms BamHI |
| NcoI: | Schnittstelle des Restriktionsenzyms NcoI |
| XbaI: | Schnittstelle des Restriktionsenzyms XbaI |
| SacI: | Schnittstelle des Restriktionsenzyms SacI |

## Patentansprüche

1. Ein isoliertes Polynukleotid, das für ein Polypeptid mit N-Succinyl-Aminoketopimelat-Transaminase Aktivität kodiert, das eine Aminosäuresequenz umfasst, die zu mindestens 90 % identisch ist mit der Sequenz von SEQ ID NO. 2.

2. Polynukleotid gemäß Anspruch 1,
**dadurch gekennzeichnet, dass** die Aminosäuresequenz zu mindestens 95 % identisch ist mit der Sequenz von SEQ ID NO. 2.

3. Polynukleotid gemäß Anspruch 1 oder 2,
**dadurch gekennzeichnet, dass** die Aminosäuresequenz identisch ist mit der Sequenz von SEQ ID NO. 2.

4. Polynukleotid gemäß Anspruch 3,
**dadurch gekennzeichnet, dass** die Nukleotidsequenz die Sequenz von Position 91 bis 1191 von SEQ ID NO. 1 umfasst.

5. Polynukleotid gemäß Anspruch 4,
**dadurch gekennzeichnet, dass** die Nukleotidsequenz die Sequenz von SEQ ID NO. 1 umfasst.

6. Ein isoliertes Polynukleotid, das komplementär ist zu den Polynukleotiden gemäß einem oder mehreren der Ansprüche 1 bis 5.

7. Ein Vektor enthaltend das Polynukleotid gemäß einem oder mehreren der Ansprüche 1 bis 6.

8. Vektor gemäß Anspruch 7,
**dadurch gekennzeichnet, dass** es sich um pXT-dapCexp handelt, hinterlegt unter der Bezeichnung DSM 13254.

9. Ein coryneformes Bakterium oder ein Bakterium der Art Escherichia coli, in das der Vektor gemäß Anspruch 7 oder 8 durch Konjugation oder Transformation überführt worden ist.

10. Ein rekombinantes coryneformes Bakterium in dem die intrazelluläre Aktivität der N-Succinyl-Aminoketopimelat-Transaminase erhöht vorliegt, wobei die genannte Erhöhung durch Überexpression eines Polynukleotids erzielt wird, das für ein Polypeptid kodiert, dessen Aminosäuresequenz zu mindestens 90% identisch ist mit der von SEQ ID NO. 2 und wobei die Überexpression durch Erhöhung der Kopienzahl oder durch Verwendung eines starken Promoters erreicht wird.

11. Coryneformes Bakterium gemäß Anspruch 10,
**dadurch gekennzeichnet, dass** die Aminosäuresequenz des Polypeptids zu mindestens 95% identisch ist mit der von SEQ ID NO. 2.

12. Coryneformes Bakterium gemäß Anspruch 11,
**dadurch gekennzeichnet, dass** die Aminosäuresequenz des Polypeptids identisch ist mit der von SEQ ID NO. 2.

13. Coryneformes Bakterium gemäß einem der Ansprüche 9 bis 12, **dadurch gekennzeichnet, dass** es sich um die Gattung Corynebacterium handelt.

14. Coryneformes Bakterium gemäß Anspruch 13,
**dadurch gekennzeichnet, dass** es sich um die Art Corynebacterium glutamicum handelt.

15. Coryneformes Bakterium gemäß einem oder mehreren der Ansprüche 9 bis 14,
**dadurch gekennzeichnet, dass** es sich um eine L-Lysin produzierende Mutante handelt.

16. Verfahren zur Herstellung von L-Lysin, **dadurch gekennzeichnet, daß** man folgende Schritte durchführt:
a) Fermentation eines coryneformen Bakteriums gemäß Anspruch 9 bis 15
b) Anreicherung des L-Lysins im Medium oder in den Zellen der Bakterien, und
c) Isolieren des L-Lysins.

17. Verfahren gemäß Anspruch 16, **dadurch gekennzeichnet, daß** man Bakterien fermentiert, in denen eines oder mehrere, der Gene, ausgewählt aus der Gruppe
17.1 das für eine feed back resistente Aspartatkinase kodierende lysC-Gen,
17.2 das für die Aspartat-Semialdehyd Dehydrogenase kodierende asd-Gen,
17.3 das für die Dihydrodipicolinat-Synthase kodierende dapA-Gen,
17.4 das für die Dihydrodipicolinat-Reduktase kodierende dapB-Gen,
17.5 das für die Tetrahydrodipicolinat-Succinylase kodierende dapD-Gen,
17.6 das für die N-Succinyl-Diaminopimelat-Desuccinylase kodierende dapE-Gen,
17.7 das für die Diaminopimelat-Epimerase kodierende dapF-Gen,
17.8 das für die Diaminopimelat-Decarboxylase kodierende lysA-Gen,
17.9 das für die Diaminopimelat-Dehydrogenase kodierende ddh-Gen,
17.10 das für den Lysin-Export kodierende lysE-Gen,
17.11 das für die Pyruvat Carboxylase kodierende pyc-Gen,
17.12 das für die Malat-Chinon-Oxidoreduktase kodierende mqo-Gen,
17.13 das zwal-Gen
17.14 das für die Glutamatdehydrogenase kodierende gdh-Gen
überexprimiert wird.

18. Aus coryneformen Bakterien stammende DNA, die für ein N-Succinyl-Aminoketopimelat-Transaminase Enzymprotein kodiert, dessen Aminosäuresequenz (SEQ ID NO. 2) an Position 209 jede andere Aminosäure ausgenommen L-Prolin enthält.

19. DNA gemäß Anspruch 18 **dadurch gekennzeichnet, daß** diese für ein N-Succinyl-Aminoketopimelat-Transaminase Enzymprotein kodiert, dessen Aminosäuresequenz an Position 209 L-Leucin enthält, dargestellt in SEQ ID NO. 4.

20. DNA gemäß Anspruch 19 **dadurch gekennzeichnet, daß** diese an Position 716 die Nukleobase Thymin enthält, dargestellt in SEQ ID NO. 3.

21. Rekombinante coryneforme Bakterien die eine DNA gemäß Anspruch 18, 19 oder 20 enthalten.

22. Verfahren zur Herstellung von L-Lysin gemäß Anspruch 16, **dadurch gekennzeichnet, daß** man in Schritt a) die Bakterien gemäß Anspruch 21 einsetzt.

## Claims

1. Isolated polynucleotide which codes for a polypeptide having N-succinyl-aminoketopimelate transaminase activity and comprising an amino acid sequence which is at least 90% identical to the sequence of SEQ ID NO. 2.

2. Polynucleotide according to Claim 1, **characterized in that** the amino acid sequence is at least 95% identical to the sequence of SEQ ID NO. 2.

3. Polynucleotide according to Claim 1 or 2, **characterized in that** the amino acid sequence is identical to the sequence of SEQ ID NO. 2.

4. Polynucleotide according to Claim 3, **characterized in that** the nucleotide sequence comprises the sequence from position 91 to position 1191 of SEQ ID NO. 1.

5. Polynucleotide according to Claim 4, **characterized in that** the nucleotide sequence comprises the sequence of SEQ ID NO. 1.

6. Isolated polynucleotide which is complementary to the polynucleotides according to one or more of Claims 1 to 5.

7. Vector comprising the polynucleotide according to one or more of Claims 1 to 6.

8. Vector according to Claim 7, **characterized in that** it is pXT-dapCexp, deposited under the name DSM 13254.

9. Coryneform bacterium or bacterium of the species *Escherichia coli,* into which the vector according to Claim 7 or 8 has been transferred by conjugation or transformation.

10. Recombinant coryneform bacterium which has an increased intracellular activity of N-succinyl-aminoketopimelate transaminase, said increase being achieved by overexpressing a polynucleotide coding for a polypeptide whose amino acid sequence is at least 90% identical to that of SEQ ID NO. 2, and said overexpression being attained by increasing the copy number or by using a strong promoter.

11. Coryneform bacterium according to Claim 10, **characterized in that** the amino acid sequence of the polypeptide is at least 95% identical to that of SEQ ID NO. 2.

12. Coryneform bacterium according to Claim 11, **characterized in that** the amino acid sequence of the polypeptide is identical to that of SEQ ID NO. 2.

13. Coryneform bacterium according to any of Claims 9 to 12, **characterized in that** the genus is *Corynebacterium.*

14. Coryneform bacterium according to Claim 13, **characterized in that** the species is *Corynebacterium glutamicum.*

15. Coryneform bacterium according to one or more of Claims 9 to 14, **characterized in that** it is an L-lysine-producing mutant.

16. Process for the preparation of L-lysine, **characterized in that** the following steps are carried out:
a) fermentation of a coryneform bacterium according to Claims 9 to 15;
b) concentration of L-lysine in the medium or in the bacterial cells; and
c) isolation of L-lysine.

17. Process according to Claim 16, **characterized in that** bacteria, in which one or more of the genes, selected from the group consisting of
17.1 the lysC gene coding for a feedback-resistant aspartate kinase,
17.2 the asd gene coding for aspartate-semi-aldehyde dehydrogenase,
17.3 the dapA gene coding for dihydrodipicolinate synthase,
17.4 the dapB gene coding for dihydrodipicolinate reductase,
17.5 the dapD gene coding for tetrahydrodipicolinate succinylase,
17.6 the dapE gene coding for N-succinyl-diaminopimelate desuccinylase,
17.7 the dapF gene coding for diaminopimelate epimerase,
17.8 the lysA gene coding for diaminopimelate decarboxylase,
17.9 the ddh gene coding for diaminopimelate dehydrogenase,
17.10 the lysE gene coding for lysine export,
17.11 the pyc gene coding for pyruvate carboxylase,
17.12 the mqo gene coding for malate-quinone oxidoreductase,
17.13 the zwa1 gene,
17.14 the gdh gene coding for glutamate dehydrogenase,
are overexpressed, are subjected to fermentation.

18. DNA derived from coryneform bacteria, which codes for an N-succinyl-aminoketopimelate transaminase enzyme protein whose amino acid sequence (SEQ ID NO. 2) includes any amino acid other than L-proline in position 209.

19. DNA according to Claim 18, **characterized in that** it codes for an N-succinyl-aminoketopimelate transaminase enzyme protein whose amino acid sequence includes L-leucine in position 209, depicted in SEQ ID NO. 4.

20. DNA according to Claim 19, **characterized in that** it includes the nucleobase thymine in position 716, depicted in SEQ ID NO. 3.

21. Recombinant coryneform bacteria comprising a DNA according to Claim 18, 19 or 20.

22. Process for the preparation of L-lysine according to Claim 16, **characterized in that** the bacteria according to Claim 21 are employed in step a).

## Revendications

1. Polynucléotide isolé, qui code pour un polypeptide à activité N-succinyl-aminocétopimélate transaminase comprenant une séquence d'aminoacides qui est identique à raison d'au moins 90 % à la séquence de SEQ ID n° 2.

2. Polynucléotide selon la revendication 1, **caractérisé en ce que** la séquence d'aminoacides est identique à raison d'au moins 95 % à la séquence de SEQ ID n° 2.

3. Polynucléotide selon la revendication 1 ou 2, **caractérisé en ce que** la séquence d'aminoacides et identique à la séquence de SEQ ID n° 2.

4. Polynucléotide selon la revendication 3, **caractérisé en ce que** la séquence nucléotidique comprend la séquence de la position 91 à la position 1191 de SEQ ID n° 1.

5. Polynucléotide selon la revendication 4, **caractérisé en ce que** la séquence nucléotidique comprend la séquence de SEQ ID n° 1.

6. polynucléotide isolé, qui est complémentaire des polynucléotides selon une ou plusieurs des revendications 1 à 5.

7. Vecteur contenant le polynucléotide selon une ou plusieurs des revendications 1 à 6.

8. Vecteur selon la revendication 7, **caractérisé en ce qu'**il s'agit de pXT-dapCexp, déposé sous le numéro d'identification DSM 13254.

9. Bactérie corynéforme ou bactérie appartenant à l'espèce *Escherichia coli,* dans laquelle le vecteur selon la revendication 7 ou 8 a été intégré par conjugaison ou transformation.

10. Bactérie corynéforme recombinante, dans laquelle l'activité intracellulaire de la N-succinyl-aminocétopimélate transaminase est présente augmentée, ladite augmentation étant réalisée par surexpression d'un polynucléotide codant pour un polypeptide dont la séquence d'aminoacides est identique à raison d'au moins 90 % à celle de SEQ ID n° 2 et la surexpression étant réalisée par augmentation du nombre de copies ou par utilisation d'un promoteur fort.

11. Bactérie corynéforme selon la revendication 10, **caractérisée en ce que** la séquence d'aminoacides du polypeptide est identique à raison de 95 % à celle de SEQ ID n° 2.

12. Bactérie corynéforme selon la revendication 11, **caractérisée en ce que** la séquence d'aminoacides du polypeptide est identique à celle de SEQ ID n° 2.

13. Bactérie corynéforme selon l'une quelconque des revendications 9 à 12, **caractérisée en ce qu'**il s'agit du genre *Corynebacterium.*

14. Bactérie corynéforme selon la revendication 13, **caractérisée en ce qu'**il s'agit de l'espèce *Corynebacterium glutamicum.*

15. Bactérie corynéforme selon une ou plusieurs des revendications 9 à 14, **caractérisée en ce qu'**il s'agir d'un mutant produisant de la L-lysine.

16. Procédé pour la production de L-lysine, **caractérisé en ce qu'**on effectue les étapes suivantes :
a) culture par fermentation d'une bactérie corynéforme selon l'une quelconque des revendications 9 à 15
b) accumulation de la L-lysine dans le milieu ou dans les cellules des bactéries, et
c) isolement de la L-lysine.

17. Procédé selon la revendication 16, **caractérisé en ce qu'**on cultive par fermentation des bactéries chez lesquelles est/sont surexprimé(s) un ou plusieurs des gènes choisis dans le groupe constitué par
17.1 le gène lysc codant pour une aspartate kinase résistante à la rétro-régulation,
17.2 le gène asd codant pour l'aspartate-semi-aldéhyde déshydrogénase,
17.3 le gène depA codant pour la dihydrodipicolinate synthase,
17.4 le gène dapB codant pour la dihydrodipicolinate réductase,
17.5 le gène dapD codant pour la tétrahydrodipicolinate succinylase,
17.6 le gène dapE codant pour la N-succinyl-diaminopimélate désuccinylase,
17.7 le gène dapF codant pour la diaminopimélate épimérase,
17.8 le gène lysA codant pour la diaminopimélate décarboxylase,
17.9 le gène ddh codant pour la diaminopimélate déshydrogénase,
17.10 le gène lysE codant pour l'export de lysine,
17.11 le gène pyc codant pour la pyruvate carboxylase,
17.12 le gène mqo codant pour la malate-quinone oxydoréductase,
17.13 le gène zwal,
17.14 le gène gdh codant pour la glutamate déshydrogénase.

18. ADN provenant de bactéries corynéformes, qui code pour une protéine enzyme N-succinyl-aminocétopimélate transaminase, dont la séquence d'aminoacides (SEQ ID n° 2) contient à la position 209 tout autre aminoacide hormis la L-proline.

19. ADN selon la revendication 18, **caractérisé en ce qu'**il code pour une protéine enzyme N-succinyl-aminocétopimélate transaminase dont la séquence d'aminoacides contient à la position 209 la L-leucine, représentée dans SEQ ID n° 4.

20. ADN selon la revendication 19, **caractérisé en ce qu'**il contient à la position 716 la base nucléique thymine, représenté dans SEQ ID n° 3.

21. Bactéries corynéformes recombinantes, qui contiennent un ADN selon la revendication 18, 19 ou 20.

22. Procédé pour la production de L-lysine selon la revendication 16, **caractérisé en ce que** dans l'étape a) on utilise les bactéries selon la revendication 21.
